# EUROPEAN PATENT APPLICATION

(11) **EP 4 332 200 A1**
(43) Date of publication of application: **06.03.2024**
(21) Application number: 22842075.8
(22) Date of filing: 11.07.2022
(51) Int. Cl.: C10G 2/00, C10G 3/00, C10J 3/00, C25B 3/07, C25B 3/26

(54) **SYNTHETIC FUEL PRODUCTION METHOD**

(30) Priority: 12.07.2021 JP 2021115085
(71) Applicant: Toyo Engineering Corporation, Tokyo 105-0003 (JP)
(72) Inventor: KAMIYAMA, Keita, Narashino-shi, Chiba 275-0024 (JP)
(74) Representative: Gille Hrabal Partnerschaftsgesellschaft mbB Patentanwälte
(86) International application number: PCT/JP2022/027233
(87) International publication number: WO 2023/286731

(57) **Abstract**

An amount of atmospheric emission of carbon dioxide can be reduced by a method for producing a synthetic fuel including a gasification step G of gasifying waste by reacting it with oxygen and water at a high temperature, a carbon dioxide separation step S of separating carbon dioxide from a gasified gas G1 produced in the step G and an FT synthesis step FT of producing the synthetic fuel by Fischer-Tropsch synthesis from a synthetic gas G2 from which carbon dioxide has been separated in the step S, the method for producing a synthetic fuel further including a carbon dioxide electrolysis step E of electrolyzing the carbon dioxide separated in the step S to produce an electrolyzed gas G3 containing carbon monoxide and carbon dioxide and a methanol synthesis step M of reacting the electrolyzed gas G3 produced in the step E with hydrogen to produce methanol.

## Description

### Field of the Invention

The present invention relates to a method for producing a synthetic fuel such as SAF (sustainable aviation fuel), a diesel fuel or the like from waste such as biomass or the like, and more specifically relates to a method for producing a synthetic fuel that can reduce an amount of atmospheric emission of carbon dioxide produced when gasifying waste by reacting the waste, oxygen and water at a high temperature.

### Background of the Invention

Conventionally, there has been known a technology of gasifying waste such as woody biomass, MSW (municipal solid waste) or the like by reacting the waste, oxygen and water in a gasification furnace at a high temperature, and from carbon monoxide and hydrogen thus obtained, producing a synthetic fuel by Fischer-Tropsch (FT) synthesis.

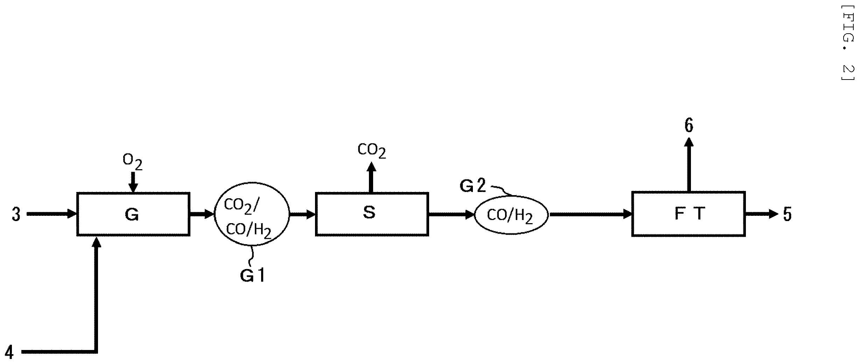

FIG. 2 is a diagram illustrating an example of a process flow of a conventional method for producing a synthetic fuel. This method illustrated in FIG. 2 includes a gasification step G of gasifying waste 3 such as woody biomass, MSW or the like by reacting the waste, oxygen and water 4 at a high temperature to produce a gasified gas G1 containing carbon dioxide, carbon monoxide and hydrogen, a carbon dioxide separation step S of separating carbon dioxide from the gasified gas G1 produced in the gasification step G and an FT synthesis step FT of producing a synthetic fuel by performing FT synthesis using a synthetic gas G2 (gas containing carbon monoxide and hydrogen) from which carbon dioxide has been separated in the carbon dioxide separation step S. In this conventional method, the carbon dioxide separated in the carbon dioxide separation step S is usually emitted into the atmosphere.

Note that examples of a method for producing a synthetic fuel using carbon dioxide as one of raw materials include a method described in patent literature 1. This patent literature 1 discloses a process including converting carbon dioxide and water to carbon monoxide and hydrogen by performing co-electrolysis in a syngas production cell (solid oxide electrolyzer cell), and then converting this to a hydrocarbon fuel in a catalytic reactor.

Main reactions when producing a synthetic fuel such as SAF or the like by FT synthesis from waste such as biomass or the like are expressed by the following reaction formulas:

CₚH_{q} + pH₂O -> pCO + (p + (q/2))H₂ (1)

CO + H₂O <--> CO₂ + H₂ (2)

nCO + (2n + 1)H₂ -> CₙH_{2n + 2} + nH₂O (3)

The above reaction formula (1) represents a reaction of producing carbon monoxide (CO) and hydrogen gas (H₂) by carrying out partial combustion or steam gasification of waste. Further, the reaction formula (3) represents a reaction of producing a synthetic fuel (CₙH_{2n +2}) from carbon monoxide (CO) and hydrogen gas (H₂), where a use amount of hydrogen gas (H₂) is twice or more (2n + 1) a use amount (n) of carbon monoxide (CO). On the other hand, as represented by the reaction formula (2), a shift reaction occurs between carbon monoxide and water (CO + H₂O) and carbon dioxide and hydrogen gas (CO₂ + H₂). As a result, a production amount of hydrogen gas (H₂) decreases when a production amount of carbon monoxide (CO) increases, and in reverse, a production amount of carbon monoxide (CO) decreases when a production amount of hydrogen gas (H₂) increases. Therefore, when a production amount of hydrogen gas (H₂) is increased to twice or more a production amount of carbon monoxide, a production amount of carbon monoxide (CO) decreases for that. In addition, a production amount of carbon dioxide (CO₂) emitted into the atmosphere increases.

Further, patent literature 2 discloses a process including producing hydrogen using renewable energy, producing methanol utilizing the produced hydrogen and carbon dioxide recovered from an exhaust gas and converting the methanol to gasoline.

Further, patent literature 3 discloses a method for producing methanol from a produced gas produced by gasifying biomass. In this method, hydrogen gas generated through electrolysis of water is supplied such that the amount of hydrogen gas is adjusted to at least twice the amount of carbon monoxide contained in the produced gas.

### Prior Art Documents

### Patent Literatures

Patent Literature 1: JP-A 2016-511296
Patent Literature 2: US-A 2009-0289227
Patent Literature 3: JP-A 2002-193858

### Summary of the Invention

### Problem to be solved by the Invention

In the conventional method illustrated in FIG. 2, the carbon dioxide separated in the carbon dioxide separation step S is usually emitted into the atmosphere. However, atmospheric emission of large amounts of carbon dioxide, which is one of the greenhouse gases, is not preferable from the viewpoint of preventing global warming. Therefore, in order to reduce an amount of atmospheric emission of carbon dioxide, the present inventor studied an effective method for recycling carbon dioxide.

In other words, an objective of the present invention is to provide a method for producing a synthetic fuel that can reduce an amount of atmospheric emission of carbon dioxide. Further, another objective of the present invention is to provide a method for producing also methanol or gasoline together with a synthetic fuel.

### Means to solve the Problem

As a result of diligent study to achieve the above objectives, the present inventor found it very effective to combine a step such as a carbon dioxide electrolysis step or the like with the conventional method illustrated in FIG. 2 to recycle carbon monoxide produced by electrolysis as a raw material for methanol synthesis and further preferably synthesize gasoline from this methanol, coming to complete the present invention. In other words, the present invention includes each aspect below.

[1] A method for producing a synthetic fuel including:
   a gasification step of gasifying waste by reacting the waste, oxygen and water at a high temperature to produce a gasified gas containing carbon dioxide, carbon monoxide and hydrogen;
   a carbon dioxide separation step of separating carbon dioxide from the gasified gas produced in the gasification step; and
   an FT synthesis step of producing the synthetic fuel by Fischer-Tropsch synthesis from a synthetic gas from which carbon dioxide has been separated in the carbon dioxide separation step,
   characterized in that the method for producing a synthetic fuel further includes:
      a carbon dioxide electrolysis step of electrolyzing the carbon dioxide separated in the carbon dioxide separation step to produce an electrolyzed gas containing carbon monoxide and carbon dioxide; and
      a methanol synthesis step of reacting the electrolyzed gas produced in the carbon dioxide electrolysis step with hydrogen to produce methanol.
[2] The method for producing a synthetic fuel according to [1], further including a gasoline production step of producing gasoline by an MTG method from the methanol produced in the methanol synthesis step.
[3] The method for producing a synthetic fuel according to [1] or [2], further including a water electrolysis step of electrolyzing water to produce oxygen and hydrogen, the produced hydrogen being supplied to the methanol synthesis step and the produced oxygen being supplied to the gasification step.
[4] The method for producing a synthetic fuel according to any of [1] to [3], further including an oxygen separation step of separating oxygen from air, the separated oxygen being supplied to the gasification step.
[5] A method for improving a synthetic fuel production facility to reduce an amount of atmospheric emission of carbon dioxide generated in an apparatus of an existing synthetic fuel production facility,
   the existing synthetic fuel production facility including:
   a gasification apparatus for gasifying waste by reacting the waste, oxygen and water at a high temperature to produce a gasified gas containing carbon dioxide, carbon monoxide and hydrogen;
   a carbon dioxide separation apparatus for separating carbon dioxide from the gasified gas produced in the gasification apparatus; and
   an FT synthesis apparatus for producing a synthetic fuel by Fischer-Tropsch synthesis from a synthetic gas from which carbon dioxide has been separated in the carbon dioxide separation apparatus,
   the improvement method being characterized by adding to the facility, a carbon dioxide electrolysis apparatus for electrolyzing the carbon dioxide separated in the carbon dioxide separation apparatus to produce an electrolyzed gas containing carbon monoxide and carbon dioxide, and
   a methanol synthesis apparatus for reacting the electrolyzed gas produced in the carbon dioxide electrolysis apparatus with hydrogen to produce methanol.
[6] The method for improving a synthetic fuel production facility according to [5], further adding a gasoline production apparatus for producing gasoline by an MTG method from the methanol produced in the methanol synthesis apparatus.
[7] The method for improving a synthetic fuel production facility according to [5] or [6], further adding a water electrolysis apparatus for electrolyzing water to produce oxygen and hydrogen, the produced hydrogen being supplied to the methanol synthesis apparatus and the produced oxygen being supplied to the gasification apparatus.

### Effect of the Invention

In the present invention, an amount of atmospheric emission of carbon dioxide can be reduced as carbon dioxide emitted into the atmosphere in the conventional method is partially reduced to carbon monoxide in the carbon dioxide electrolysis step and this is recycled as a raw material for methanol synthesis.

Further, hydrogen produced by electrolyzing water is preferably supplied to the methanol synthesis step. This can supplement hydrogen in the raw material gases in methanol synthesis. In this case, a molar amount of the hydrogen produced by electrolyzing water and supplied to the methanol synthesis step is preferably twice or more a molar amount of carbon monoxide in the electrolyzed gas. This achieves a suitable compositional balance between the raw material gases in the methanol synthesis step.

Further, it is also preferable to supply oxygen produced by electrolyzing water to the gasification step. Utilizing this oxygen for gasifying waste can reduce load on the oxygen separation step.

### Brief Description of the Drawings

[FIG. 1] A diagram illustrating an example of a process flow of a method for producing a synthetic fuel of the present invention.
[FIG. 2] A diagram illustrating an example of a process flow of a conventional method for producing a synthetic fuel.

### Embodiments of the Invention

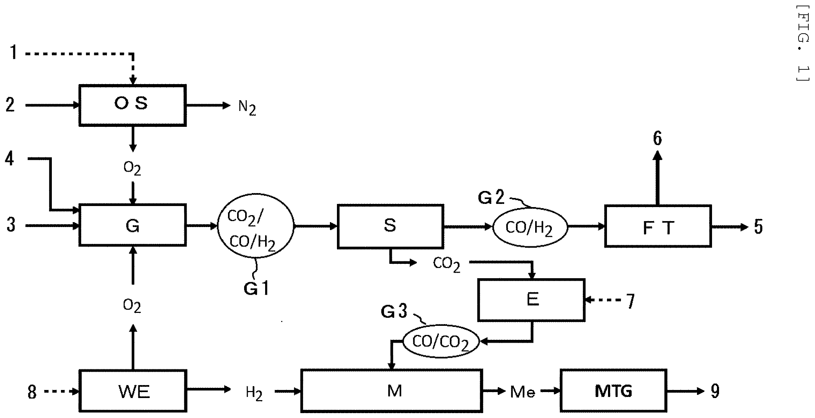

FIG. 1 is a diagram illustrating a process flow in a preferable aspect of a method for producing a synthetic fuel of the present invention. Each step is explained below. Supply of a material or electric power to each step can be carried out by providing as necessary a supply system including a supply line, and emission or removal of a product or waste from each step can be carried out by providing as necessary an emission system including an emission line.

### [Oxygen separation step]

An oxygen separation step OS shown in FIG. 1 is a step of separating oxygen from air 2. The oxygen separated in this oxygen separation step is supplied to a gasification step G described later.

Typical examples of a method for separating oxygen from air in this oxygen separation step include a method in which gases other than oxygen in the air (nitrogen or the like) are adsorbed onto adsorbents (for example, synthetic zeolite) by adjusting pressure to obtain a high-purity oxygen gas (vacuum pressure swing adsorption (VPSA) method). The adsorbed gases other than oxygen (nitrogen or the like) may be emitted into the atmosphere. As specific reaction conditions, types of adsorbents or configurations of reaction apparatuses including an oxygen separation apparatus or an oxygen separation system for that, publicly-known conditions, types and configurations relating to oxygen separation technology can be employed without any limitation.

In the present invention, it is preferable to use a VPSA process as above as one of steps for obtaining oxygen to be supplied to the gasification step G. However, the present invention is not limited thereto. It is also possible to obtain a high-purity oxygen gas by any other publicly-known methods (cryogenic separation method or the like) instead of a VPSA process and supply it to the gasification step G. It is often the case that a VPSA process is advantageous in terms of economy, but in a small plant, for example, a cryogenic separation method may be more advantageous in terms of economy.

### [Gasification step]

The gasification step G shown in FIG. 1 is a step of gasifying waste 3 by reacting the waste, oxygen and water 4 at a high temperature to produce a gasified gas G1 [CO₂/CO/H₂] containing carbon dioxide, carbon monoxide and hydrogen. The gasified gas G1 [CO₂/CO/H₂] produced in this gasification step G is supplied to a carbon dioxide separation step S described later.

Typical examples of a method for gasifying waste by reacting the waste, oxygen and water at a high temperature in this step G include a method in which the waste, oxygen and water are supplied to a gasification furnace (melting furnace) and reacted at predetermined temperature and pressure. As specific reaction conditions or configurations of reaction apparatuses including a gasification apparatus or a gasification system for that, publicly-known conditions and configurations relating to gasification technology can be employed without any limitation. For example, a reaction temperature is usually 700°C or more and preferably 800°C to 1200°C.

Examples of the waste used as a raw material in the gasification step G can include a solid and a liquid containing a gasifiable hydrocarbon component and a mixture of them, and an example is woody biomass or MSW (municipal solid waste). However, the present invention is not limited thereto. For example, waste such as herbaceous biomass, PKS (palm kernel shell), OPT (oil palm trunk) or the like can also be used.

### [Carbon dioxide separation step]

The carbon dioxide separation step S shown in FIG. 1 is a step of separating carbon dioxide from the gasified gas G1 [CO₂/CO/H₂] produced in the gasification step G.

The carbon dioxide separated in this carbon dioxide separation step S is not emitted into the atmosphere, but supplied to a carbon dioxide electrolysis step E described later and recycled. As a result, an amount of atmospheric emission of carbon dioxide can be reduced.

On the other hand, a gas after carbon dioxide has been separated, in other words, a synthetic gas G2 [CO/H₂] containing carbon monoxide and hydrogen is supplied to an FT synthesis step FT described later as a raw material of a synthetic fuel.

Typical examples of a method for separating carbon dioxide from the gasified gas G1 in this step S include a chemical absorption method including absorbing carbon dioxide by an absorption liquid such as an amine or the like in an absorption step and heating the absorption liquid to separate carbon dioxide in a regeneration step. As specific reaction conditions or configurations of reaction apparatuses including a carbon dioxide separation apparatus or a carbon dioxide separation system for that, publicly-known conditions and configurations relating to carbon dioxide separation technology can be employed without any limitation.

### [Carbon dioxide electrolysis step]

The carbon dioxide electrolysis step E shown in FIG. 1 is a step of electrolyzing the carbon dioxide separated in the carbon dioxide separation step S to produce an electrolyzed gas G3 [CO/CO₂] containing carbon monoxide and carbon dioxide. The electrolyzed gas G3 [CO/CO₂] produced in this carbon dioxide electrolysis step E is supplied to a methanol synthesis step M described later.

The carbon dioxide electrolysis step E is typically a step of partially reducing carbon dioxide to carbon monoxide by electrolysis. Accordingly, the produced electrolyzed gas G3 [CO/CO₂] is typically a mixed gas of carbon monoxide produced by reduction and unreduced carbon dioxide. As specific electrolysis conditions or configuration of electrolysis apparatus or electrolysis system for that, publicly-known conditions and configuration relating to carbon dioxide electrolysis technology can be employed without any limitation.

This step of partially reducing carbon dioxide to carbon monoxide by electrolysis also has the advantages of allowing electrolysis at a low temperature (less than 100°C) and also not causing the problem of performance degradation due to adhesion of deposited carbon to an electrode compared to a method as described in patent literature 1 (a process in which carbon dioxide and water are converted to carbon monoxide and hydrogen by performing co-electrolysis at a high temperature (500°C or more) in a solid oxide electrolyzer cell).

Further, the electrolyzed gas G3 produced in the carbon dioxide electrolysis step E is supplied to the methanol synthesis step M described later and used as a raw material of methanol.

It is preferable to use electric power 7 generated by renewable energy for the carbon dioxide electrolysis step E. Renewable energy is energy always present in the natural world such as sunlight, wind power, geothermal heat, water power or the like, and characterized by not emitting carbon dioxide when generated. Using electric power from this renewable energy for the carbon dioxide electrolysis step E agrees with the objective of the present invention to reduce an emission amount of carbon dioxide.

### [Water electrolysis step]

A water electrolysis step WE shown in FIG. 1 is a step of electrolyzing water to produce oxygen and hydrogen. As specific electrolysis conditions or configuration of electrolysis apparatus or electrolysis system in this step WE, publicly-known conditions and configuration relating to water electrolysis technology can be employed without any limitation.

In the water electrolysis step WE, the produced hydrogen is supplied to the methanol synthesis step M described later. The electrolyzed gas G3 and hydrogen are then reacted to produce methanol. A molar amount of the hydrogen produced in the water electrolysis step WE and supplied to the methanol synthesis step M is preferably twice or more a molar amount of carbon monoxide in the electrolyzed gas G3. This achieves a suitable compositional balance between the raw material gases in the methanol synthesis step M.

On the other hand, the oxygen produced in the water electrolysis step WE is supplied to the gasification step G. Utilizing this oxygen for gasifying waste can reduce load on the oxygen separation step.

It is preferable to use electric power 8 generated by renewable energy for the water electrolysis step WE as is the case with the carbon dioxide electrolysis step E explained above.

In the present invention, it is preferable to use the water electrolysis step WE explained above as one of steps for producing hydrogen to be supplied to the methanol synthesis step M. However, the present invention is not limited thereto. It is also possible to produce hydrogen by any other publicly-known methods instead of the water electrolysis step WE and supply it to the methanol synthesis step M.

### [FT synthesis step]

The FT synthesis step FT shown in FIG. 1 is a step of producing a synthetic fuel by Fischer-Tropsch (FT) synthesis from the synthetic gas G2 from which carbon dioxide has been separated in the carbon dioxide separation step S, in other words, the synthetic gas G2 [CO/H₂] containing carbon monoxide and hydrogen.

Fischer-Tropsch (FT) synthesis is a synthesis method for obtaining a synthetic fuel (gas and liquid hydrocarbon) from carbon monoxide and hydrogen by a catalytic reaction. A compound of iron or cobalt is usually used as a catalyst. As specific reaction conditions, types of catalysts or configurations of reaction apparatuses including an FT synthesis apparatus or an FT synthesis system in this FT synthesis, publicly-known conditions, types and configurations relating to FT synthesis technology can be employed without any limitation.

This FT synthesis step FT produces SAF (sustainable aviation fuel) and other synthetic fuels 5. Examples of the other synthetic fuels include, for example, kerosene, diesel oil, naphtha or the like. Further, a gas fraction produced at the time of synthesis is used as a fuel gas or burned by flare or the like and released into the atmosphere as an off-gas 6.

### [Methanol synthesis step]

The methanol synthesis step M shown in FIG. 1 is a step of reacting the electrolyzed gas G3 produced in the carbon dioxide electrolysis step E, in other words, the electrolyzed gas G3 containing carbon monoxide and carbon dioxide with hydrogen to produce methanol.

In this step M, methanol is typically produced by reacting carbon monoxide, carbon dioxide and hydrogen by a catalytic reaction. As specific reaction conditions, types of catalysts or configurations of reaction apparatuses including a methanol synthesis apparatus or a methanol synthesis system for that, publicly-known conditions, types and configurations relating to methanol synthesis technology can be employed without any limitation.

The methanol synthesis step M using the electrolyzed gas G3 containing carbon monoxide and carbon dioxide as a raw material as above also has the advantage of facilitating methanol synthesis with a common catalyst such as a copper-based catalyst or the like compared to when using only carbon dioxide as a raw material. Further, using the electrolyzed gas G3 as a raw material enables easy production of a synthetic gas of composition which satisfies the R value most suitable for methanol synthesis = (H₂ - CO₂)/(CO + CO₂) = 2 so that methanol can be synthesized efficiently.

### [MTG step]

An MTG step MTG shown in FIG. 1 is a gasoline production step of producing gasoline 9 by an MTG method (methanol to gasoline process) from the methanol produced in the methanol synthesis step M.

In this MTG step MTG, synthetic gasoline is typically produced from the methanol by a catalytic reaction. As specific reaction conditions, types of catalysts or configurations of reaction apparatuses including an apparatus for producing gasoline by an MTG method (MTG apparatus) or a system for producing gasoline by an MTG method (MTG system) for that, publicly-known conditions, types and configurations relating to gasoline synthesis technology can be employed without any limitation.

In the present invention, the MTG step MTG explained above preferably produces synthetic gasoline from the methanol. However, the present invention is not limited thereto. The methanol produced in the methanol synthesis step M may be used as a raw material of another compound or the methanol may be used as-is as a chemical product.

### [Method for improving synthetic fuel production facility]

The method for producing a synthetic fuel of the present invention explained above can be carried out by newly constructing all apparatuses that each carry out each of the steps. However, it can be carried out also by adding a carbon dioxide electrolysis apparatus and as necessary other apparatuses (for example, water electrolysis apparatus and MTG apparatus, in other words, apparatus for producing gasoline by MTG method) to an existing production facility.

In other words, a method for improving a synthetic fuel production facility of the present invention is an improvement method for reducing an amount of atmospheric emission of carbon dioxide generated in an apparatus of an existing synthetic fuel production facility.

Examples of the existing synthetic fuel production facility as a target to be improved can include, as one example, an existing synthetic fuel production facility including a gasification apparatus (g) for gasifying waste by reacting the waste, oxygen and water at a high temperature to produce the gasified gas G1 containing carbon dioxide, carbon monoxide and hydrogen, a carbon dioxide separation apparatus (s) for separating carbon dioxide from the gasified gas G1 produced in the gasification apparatus (g) and an FT synthesis apparatus for producing a synthetic fuel by Fischer-Tropsch synthesis from the synthetic gas G2 from which carbon dioxide has been separated in the carbon dioxide separation apparatus (s), the facility releasing the carbon dioxide separated in the carbon dioxide separation apparatus (s) into the atmosphere.

In the improvement method of the present invention, a carbon dioxide electrolysis apparatus (e) for electrolyzing the carbon dioxide separated in the carbon dioxide separation apparatus (s) to produce the electrolyzed gas G3 containing carbon monoxide and carbon dioxide and a methanol synthesis apparatus (m) for reacting the electrolyzed gas G3 produced in the carbon dioxide electrolysis apparatus (e) with hydrogen to produce methanol are added to the existing synthetic fuel production apparatus.

This addition of the carbon dioxide electrolysis apparatus (e) and the methanol synthesis apparatus (m) enables an effective reduction in an emission amount of carbon dioxide as carbon dioxide released into the atmosphere from the carbon dioxide separation apparatus (s) in the existing facility is utilized for methanol synthesis in such a manner that the carbon dioxide is collected to the carbon dioxide electrolysis apparatus (e) and partially converted in the carbon dioxide electrolysis apparatus (e) to carbon monoxide to produce the electrolyzed gas G3, which is converted to methanol.

Further, in this improvement method, it is also preferable to add an MTG apparatus for producing the gasoline 9 from the methanol produced in the methanol synthesis apparatus (m).

Further, in this improvement method, it is preferable to add a water electrolysis apparatus (we) for electrolyzing water to produce oxygen and hydrogen and supply the produced hydrogen to the methanol synthesis apparatus (m). In this case, a molar amount of the hydrogen produced in the water electrolysis apparatus (we) and supplied to the methanol synthesis apparatus (m) is preferably twice or more a molar amount of carbon monoxide in the electrolyzed gas G3. Further, it is also preferable to supply the oxygen produced in the water electrolysis apparatus (we) to the gasification apparatus (g).

Adding an apparatus such as the carbon dioxide electrolysis apparatus or the like to an existing production facility in this manner is advantageous in terms of facility costs compared to when newly constructing all facilities. Further, methanol can be newly produced by effectively utilizing carbon dioxide emitted in an existing production facility.

### Industrial Applicability

The present invention is very useful from the viewpoint of preventing global warming as it can reduce an amount of atmospheric emission of carbon dioxide by recycling carbon dioxide produced when producing a synthetic fuel from waste.

### Description of Reference Numerals

1: electric power
2: air
3: waste
4: water
5: fuel
6: off-gas
7: electric power generated by renewable energy
8: electric power generated by renewable energy
9: synthetic gasoline
Me: methanol
OS: oxygen separation step
G: gasification step
S: carbon dioxide separation step
FT: FT synthesis step
E: carbon dioxide electrolysis step
WE: water electrolysis step
M: methanol synthesis step
MTG: MTG step
G1: gasified gas
G2: synthetic gas
G3: electrolyzed gas

## Claims

1. A method for producing a synthetic fuel comprising:
a gasification step of gasifying waste by reacting the waste, oxygen and water at a high temperature to produce a gasified gas containing carbon dioxide, carbon monoxide and hydrogen;
a carbon dioxide separation step of separating carbon dioxide from the gasified gas produced in the gasification step; and
an FT synthesis step of producing the synthetic fuel by Fischer-Tropsch synthesis from a synthetic gas from which carbon dioxide has been separated in the carbon dioxide separation step,
**characterized in that** the method for producing a synthetic fuel further comprises:
a carbon dioxide electrolysis step of electrolyzing the carbon dioxide separated in the carbon dioxide separation step to produce an electrolyzed gas containing carbon monoxide and carbon dioxide; and
a methanol synthesis step of reacting the electrolyzed gas produced in the carbon dioxide electrolysis step with hydrogen to produce methanol.

2. The method for producing a synthetic fuel according to claim 1, further comprising a gasoline production step of producing gasoline by an MTG method from the methanol produced in the methanol synthesis step.

3. The method for producing a synthetic fuel according to claim 1 or 2, further comprising a water electrolysis step of electrolyzing water to produce oxygen and hydrogen, the produced hydrogen being supplied to the methanol synthesis step and the produced oxygen being supplied to the gasification step.

4. The method for producing a synthetic fuel according to any of claims 1 to 3, further comprising an oxygen separation step of separating oxygen from air, the separated oxygen being supplied to the gasification step.

5. A method for improving a synthetic fuel production facility to reduce an amount of atmospheric emission of carbon dioxide generated in an apparatus of an existing synthetic fuel production facility,
the existing synthetic fuel production facility comprising:
a gasification apparatus for gasifying waste by reacting the waste, oxygen and water at a high temperature to produce a gasified gas containing carbon dioxide, carbon monoxide and hydrogen;
a carbon dioxide separation apparatus for separating carbon dioxide from the gasified gas produced in the gasification apparatus; and
an FT synthesis apparatus for producing a synthetic fuel by Fischer-Tropsch synthesis from a synthetic gas from which carbon dioxide has been separated in the carbon dioxide separation apparatus,
the improvement method being **characterized by** adding to the facility, a carbon dioxide electrolysis apparatus for electrolyzing the carbon dioxide separated in the carbon dioxide separation apparatus to produce an electrolyzed gas containing carbon monoxide and carbon dioxide, and
a methanol synthesis apparatus for reacting the electrolyzed gas produced in the carbon dioxide electrolysis apparatus with hydrogen to produce methanol.

6. The method for improving a synthetic fuel production facility according to claim 5, further adding a gasoline production apparatus for producing gasoline by an MTG method from the methanol produced in the methanol synthesis apparatus.

7. The method for improving a synthetic fuel production facility according to claim 5 or 6, further adding a water electrolysis apparatus for electrolyzing water to produce oxygen and hydrogen, the produced hydrogen being supplied to the methanol synthesis apparatus and the produced oxygen being supplied to the gasification apparatus.
